(19) **Europäisches Patentamt**
**European Patent Office**
**Office européen des brevets**

(11) **EP 1 742 050 A2**

(12) **EUROPEAN PATENT APPLICATION**

(43) Date of publication:
**10.01.2007 Bulletin 2007/02**

(51) Int Cl.:
**G01N 33/28** (2006.01)

(21) Application number: **06076226.7**

(22) Date of filing: **14.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **05.07.2005 US 175059**

(71) Applicant: **Delphi Technologies, Inc.**
**Troy, Michigan 48007 (US)**

(72) Inventors:
• **Lin, Yingjie**
**El Paso, TX 79912 (US)**

• **Heremans, Joseph P.**
**Troy, MI 48084 (US)**
• **Wang, Su-Chee S.**
**Troy, MI 48098 (US)**

(74) Representative: **Denton, Michael John et al**
**Delphi European Headquarters,**
**64 avenue de la Plaine de France,**
**Paris Nord II,**
**B.P. 65059, Tremblay en France**
**95972 Roissy Charles de Gaulle Cedex (FR)**

(54) **Diesel fuel dilution level determination of diesel engine oil**

(57) A method for determining fuel dilution of diesel engine lubricating oil in a diesel engine. A first table contains a soot compensation factor (102-112), respectively, for each weight of oil selected among a predetermined range of oil weights. A second table contains fuel dilution levels (102'-112') for a plurality of predetermined compensated viscosity ratios. After determining the weight of the oil in the engine a first viscosity of the oil at a first temperature and a second viscosity of the oil at a second temperature are measured. Next, either a ratio (116) or a difference (714) of the first and second viscosities is determined. Using the ratio, soot in the oil is compensated using a soot compensation factor of the first table which is respective of the oil to thereby provide a compensated viscosity ratio (220). Finally, the compensated viscosity ratio is compared with the second table to thereby determine the fuel dilution level.

Fig.4.

**EP 1 742 050 A2**

**Description**

TECHNICAL FIELD

The present invention relates to detecting diesel engine lubricating oil contamination and, more particularly, to a method of detecting diesel fuel dilution levels of diesel engine lubricating oil used in diesel engines.

BACKGROUND OF THE INVENTION

Many design modifications have been applied to diesel engines, hereinafter referred to as engines, for the purpose of reducing the emission levels and improving fuel efficiency. One possible way to reduce the $NO_x$ in the exhaust gas is to post-inject a small amount of diesel fuel, hereinafter referred to simply as "fuel", into the cylinder, called post-injection. Because diesel fuel is very fluid, under several malfunction conditions of the diesel engine, hereinafter referred to simply as an "engine", for example imperfect combustion and piston ring blow-by, the fuel can pass through the piston seals and get into the engine lubricating oil, hereinafter referred to simply as "oil", thereby contaminating the oil, herein referred to as "fuel contaminated oil".

The process of fuel (diesel fuel) mixing with the oil (engine lubricating oil) resulting in fuel contaminated oil is herein referred to as "fuel dilution". The amount of fuel dilution, or the fuel dilution level, may be expressed as a percent defined as the fraction of the amount of fuel, by volume or weight, preferably, by volume, with respect to the total amount of the fuel contaminated oil, by volume or weight, preferably by volume. For example, a fuel dilution of 5%, or a fuel dilution level of 5%, of 1000 ml of a fuel contaminated oil consists of 50 ml of fuel and 950 ml of oil. After the fuel contaminates the oil, the fuel contaminated oil's viscosity is lowered compared to the oil's (non-fuel contaminated) viscosity at a given temperature. At a given fuel dilution level, for example 7% to 10%, the fuel contaminated oil starts to lose its lubricating qualities, especially at high temperature, about 100 degrees C or greater, whereupon engine parts can become stressed enough to result in untoward consequences, as for example the bearings failing. Post-injection thus requires a method to measure the fuel dilution level in order to alert the driver to change the oil to avoid engine failure.

Accordingly, what is needed in the art is a method to determine the diesel fuel dilution level in order to alert the driver to change the oil to avoid engine failure.

SUMMARY OF THE INVENTION

The present invention is a method to measure the diesel fuel dilution level of fuel contaminated diesel engine lubricating oil utilizing viscosity information in conjunction with engine lubricating oil soot contamination information.

Most current oils are multi-weighted. Above, approximately, 100 degrees C viscosity modifiers in multi-weighted oils become active. The present invention utilizes temperatures at or below, approximately, 100 degrees C such that viscosity modifiers in multi-weighted oils do not become active.

It is known in the art that viscosity changes with temperature in a predetermined temperature range, for example from 60 degrees C to 100 degrees C, are different for different weights of oil. The reason for this is that the temperature dependence of the viscosity of a fluid, such as oil, follows an activated behavior (an Arrhenius plot) with an activation energy that is a fixed fraction of the heat of vaporization (W. J. Moore, Physical Chemistry, Addison-Wesley, 1964) which in turn is a function of the molecular weight of the fluid. Thus, a very different temperature dependence of viscosity between fuel and oil is also expected. After fuel is added into oil, the viscosity of the fuel contaminated oil drops, but since the viscosity of the fuel contaminated oil is also a function of the weight of the oil, a measurement of the viscosity of the fuel contaminated oil alone cannot be used as an indicator of fuel dilution levels. Furthermore, viscosity changes by fuel dilution are smaller at higher temperatures and larger at lower temperatures. For example, at 60 degrees C pure, fresh 15W40 oil having a viscosity of 47cst drops to a viscosity of 38cst with a fuel dilution level of 5%, whereas at 100 degrees C pure, fresh 15W40 oil having a viscosity of 14.7cst drops to 12.6cst 38cst with a fuel dilution level of 5%. Thus, viscosity measurements are less sensitive to fuel dilution levels as the temperature is increased.

There is an additional issue specific to the oil: the diesel combustion process generates soot that can get into the oil, thereby producing a soot contaminated oil. The soot contaminated oil's viscosity is increased compared to the oil's (non-soot contaminated) viscosity at a given temperature. The amount of soot contamination, or soot concentration, can be expressed as a percentage in an analogous manner as previously described for fuel dilution. The impact of increased soot concentration on the viscosity of the oil is similar of that of fuel dilution, but in the opposite direction. In general, the oil can be simultaneously contaminated with fuel and soot, herein referred to as "fuel-soot oil". Thus, a measurement of viscosity of fuel-soot oil alone can give a false sense of security: the fuel-soot oil can display no variation in viscosity while having lost its lubricating quality if soot and fuel are simultaneously present. This could conceivably be addressed by looking at the fuel-soot oil viscosity's temperature dependence, but experimentation shows that fuel dilution reduces the viscosity ratio (lower temperature viscosity/higher temperature viscosity) of fuel contaminated oil or fuel-soot oil while soot increases the viscosity ratio of soot contaminated oil or fuel-soot oil.

One method to determine oil viscosity is given in United States Patent 6,810,717 B2, issued on November 2, 2004, and assigned to the assignee hereof, the entire disclosure of which is hereby incorporated herein by reference. One method to determine soot content in diesel engine oil is given in United States Patent Application Publication No. US 2004/0036487 A1, assigned to the assignee hereof, the entire disclosure of which is hereby incorporated herein by reference.

In a first preferred embodiment of the present invention, the fuel dilution level is determined through a soot compensated viscosity ratio for a given weight of oil.

It is well known in the art, theoretically and empirically, that with fresh, clean oil in the engine, the ratio of viscosity at a first temperature to the viscosity at a second temperature, wherein the first temperature is, preferably, lower than the second temperature, for example, a first temperature of 60 degrees C and a second temperature of 100 degrees C, uniquely determines the weight of the oil. Alternatively, by definition, the weight of the oil is the viscosity at a predetermined temperature, for example 40 degrees C, and can be determined, therefore, by measuring the viscosity at the predetermined temperature. To be described later, a unique soot compensation factor can be empirically determined for a given weight of oil, whereby different weights of oil have different unique soot compensation factors independent of fuel dilution levels, which are then stored in a first look-up table. The unique soot compensation factor for a given weight of oil is used to produce a compensated viscosity ratio from the measurements of the viscosity at the first and second temperatures, whereby the fuel dilution level can be ascertained from a second look-up table containing fuel dilution levels for various compensated viscosity ratios which are unique for the given weight of oil, wherein different weights of oil have unique entries of fuel dilution levels for various compensated viscosity ratios for each weight of oil stored in the second look-up table.

In a second preferred embodiment of the present invention, the fuel dilution level is determined through a soot compensated viscosity difference for a given weight of oil.

By definition, the weight of the oil is the viscosity at a predetermined temperature, for example 40 degrees C, and can be determined, therefore, by measuring the viscosity at the predetermined temperature. To be described later, a unique soot compensation factor can be empirically determined for a given weight of oil, whereby different weights of oil have different unique soot compensation factors independent of fuel dilution levels which are then stored in a third look-up table. The unique soot compensation factor for a given weight of oil is used to produce a compensated viscosity difference from the measurements of the viscosity at the first and second temperatures, whereby the fuel dilution level can be ascertained from a fourth look-up table containing fuel dilution levels for various compensated viscosity differences which are unique for the given weight of oil, wherein different weights of oil have unique entries of fuel dilution levels for various compensated viscosity differences for each weight of oil stored in the fourth look-up table.

Many variations in the embodiments of present invention are contemplated as described hereinbelow in more detail. Other applications of the present invention will become apparent to those skilled in the art when the following description of the best mode contemplated for practicing the invention is read in conjunction with the accompanying drawings.

BRIEF DESCRIPTION OF THE DRAWINGS

The description herein makes reference to the accompanying drawings, wherein like reference numerals refer to like parts throughout the several views.

Figure 1 is a graph of plots of viscosity ratios versus soot percentages for various fuel dilution levels.
Figure 2 is a graph of plots of soot compensated viscosity ratios versus soot percentages for various fuel dilution levels.
Figure 3 is a graph of plots of fuel dilution levels versus average soot compensated viscosity ratios.
Figure 4 is a graph of plots of calculated fuel dilution levels versus actual fuel dilution levels.
Figure 5 is a graph of plots of viscosity differences versus actual fuel dilution levels for various soot percentages.
Figure 6 is a graph of plots of ratios of viscosity differences versus actual fuel dilution levels for various soot percentages.
Figure 7 is a graph of plots of soot compensated viscosity differences versus actual fuel dilution levels for various soot percentages.
Figure 8 is a graph of plots of calculated fuel dilution levels versus actual fuel dilution levels.

DESCRIPTION OF THE PREFERRED EMBODIMENT

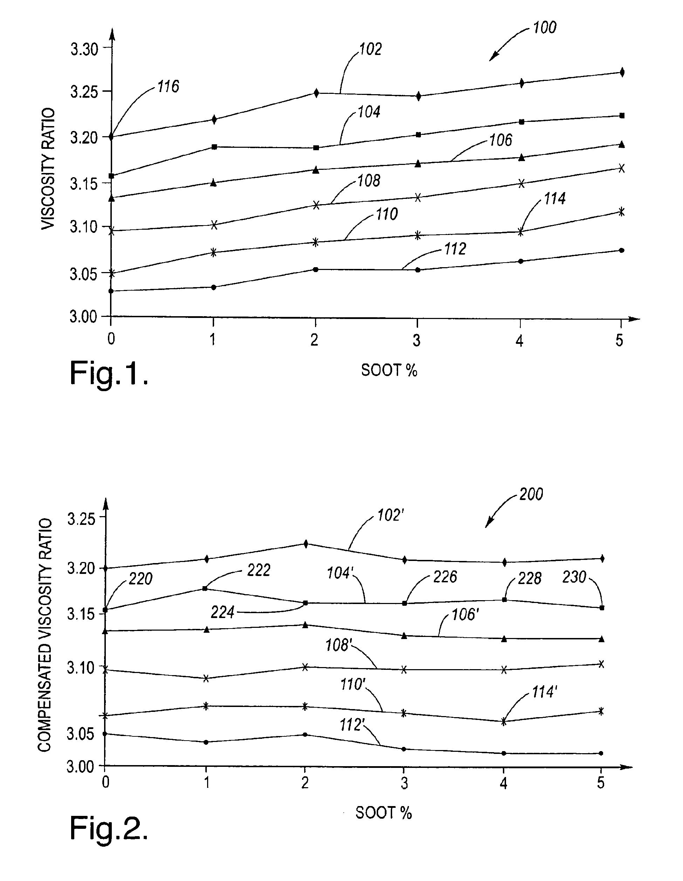

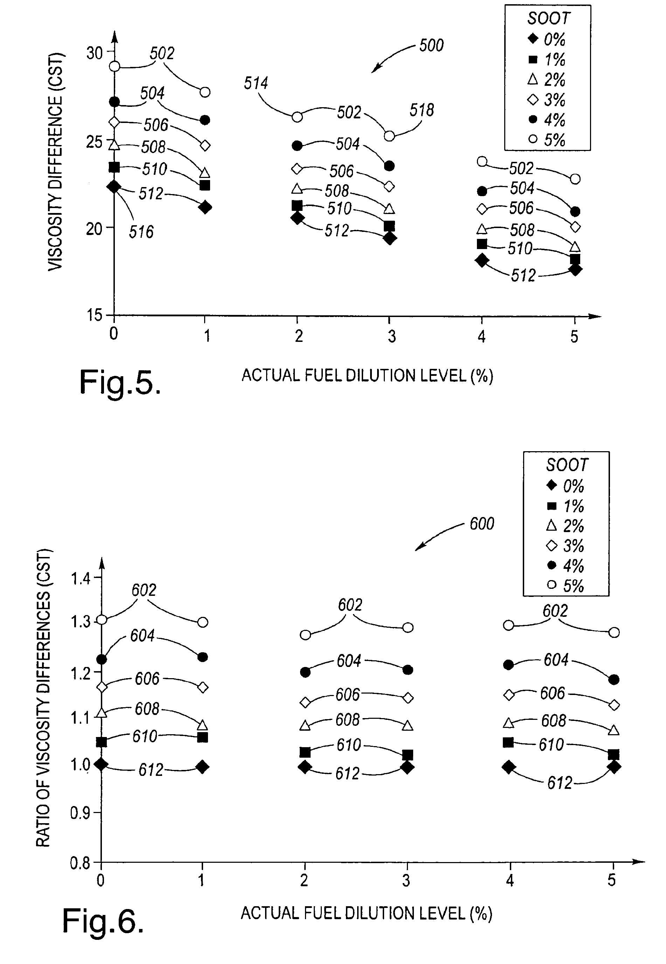

By example, 15W40 oil is used in the plots of Figures 1 through 9. Figures 1 through 4 pertain to the first preferred embodiment of the present invention, wherein fuel dilution level is determined through a soot compensated viscosity ratio for a given weight of oil. Figures 5 through 8 pertain to the second preferred embodiment of the present invention, wherein fuel dilution level is determined through a soot compensated viscosity difference for a given weight of oil.

Referring firstly to the first preferred embodiment of the present invention, Figure 1 is a graph 100 of plot curves 102 through 112 of viscosity ratios versus soot percentages for various fuel dilution levels wherein each curve corresponds to a particular fuel dilution level and each viscosity ratio is the ratio of the viscosity at 60 degrees C to the viscosity at 100 degrees C at a particular soot percentage and a particular fuel dilution level. Curve 102 corresponds to a fuel dilution level of 0% (ie., zero percent). Curve 104 corresponds to a fuel dilution level of 1%. Curve 106 corresponds to a fuel dilution level of 2%. Curve 108 corresponds to a fuel dilution level of 3%. Curve 110 corresponds to a fuel dilution level of 4%. Curve 112 corresponds to a fuel dilution level of 5%. The curves 102 through 112 show that soot contamination (soot %) has the same effect on viscosity ratios independent of the fuel dilution level since the slopes of the curves are almost the same. Therefore, the viscosity ratios can be compensated for the error introduced by soot contamination by using the soot percent (ie., soot %) level to do the compensation.

Figure 2 is a graph 200 of curves 102' through 112' of soot compensated viscosity ratios (compensated viscosity ratios). In this regard, each curve 102' through 112' corresponds to a respective curve 102 through 112 of Figure 1, as identified by priming, wherein: curve 102' corresponds to a fuel dilution level of 0%; curve 104' corresponds to a fuel dilution level of 1%; curve 106' corresponds to a fuel dilution level of 2%; curve 108' corresponds to a fuel dilution level of 3%; curve 110' corresponds to a fuel dilution level of 4%; and curve 112' corresponds to a fuel dilution level of 5%. Each viscosity ratio of Figure 1 may be soot compensated to yield a soot compensated viscosity ratio of Figure 2 by subtracting the product of the average slope of the curves 102 through 112 and the soot percent at that viscosity ratio from the viscosity ratio.

For example, the average slope of the curves 102 through 112 of Figure 1 is, numerically, approximately 0.012, which is the soot compensation factor. Point 114 of Figure 1 has a viscosity ratio of, approximately, 3.095 at 4% soot and a fuel dilution level of 4%. The product of 0.012 (ie., the soot compensation factor) times 4 (ie., the soot percent) is 0.048 (ie., a compensation value). Subtracting 0.048 (ie., the compensation value) from 3.095 (ie., the viscosity ratio) yields 3.047, which is the soot compensated viscosity ratio at the point 114' of Figure 2 and which corresponds to the viscosity ratio at the point 114 of Figure 1.

The average slope of the curves 102 through 112 is the soot compensation factor for the given weight of oil (15W40). It can be seen in Figure 2 that the soot compensated viscosity ratios have, approximately, the same value for all soot percent at a given fuel dilution level wherein the fuel dilution levels of curves 102' through 112' correspond to the fuel dilution levels of curves 102 through 112, respectively, of Figure 1.

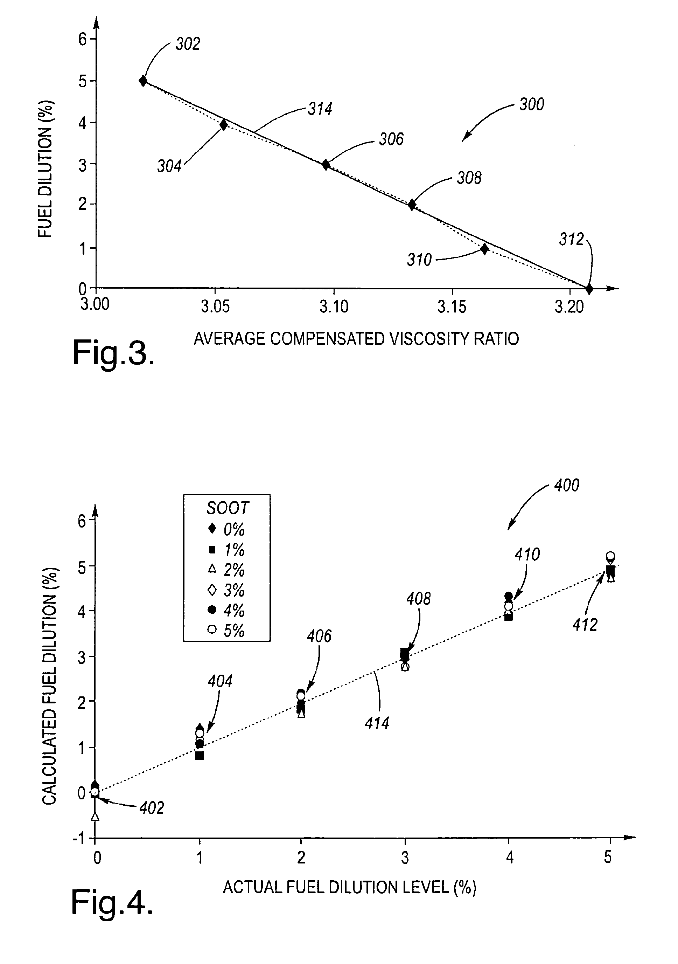

Figure 3 is a graph 300 of fuel dilution levels versus average soot compensated viscosity ratios (average compensated viscosity ratios) of Figure 2 for each particular fuel dilution level of Figure 2. Each point 302 through 312 is obtained from Figure 2 by dividing the sum of the soot compensated viscosity ratios by the number of soot compensated viscosity ratios for a given fuel dilution level. For example, point 310, having a fuel dilution of 1% at a soot compensated viscosity ratio of, approximately, 3.163 is obtained by summing the soot compensated viscosity ratios 220 through 230 of Figure 2 and dividing by 6, the number of soot compensated viscosity ratios 220 through 230 for a fuel dilution level of 1%. The straight line 314 is the best fit through the points 302 through 312.

In the example of Figure 3, the equation of the straight line 314 is:

$$Y = -26.78X + 85.84 \qquad (1)$$

where Y is the fuel dilution level in percent and X is the average soot compensated viscosity ratio.

Figure 4 is a graph of plots of calculated fuel dilution levels 402 through 412 using equation (1) versus actual fuel dilution levels. The dotted straight line 414 represents the best fit through the points 402 through 412.

The average slope of the curves 102 through 112, the soot compensation factor, and the best fit straight line 414, equation (1), are unique for the given weight of oil 15W40 in the example of the depicted figures. Each different weight of oil will have a unique soot compensation factor and unique best fit straight line. This is due to the dependence of the activation energy characterizing the temperature dependence of different fluids on their molecular weight, as previously described. For each different weight of oil of interest, the soot compensation factors and best fit straight lines can be empirically determined and stored, as for example in a vehicle sensor memory or in a vehicle microprocessor memory, in first and second look-up tables, respectively.

The first preferred embodiment of the present invention may be implemented as follows.

With fresh, clean oil in the engine, a viscosity measurement is made at a predetermined temperature, which measurement is indicative of the weight of the oil. Alternatively, a first ratio of the viscosity (first viscosity ratio) measured at 60 degrees C and at 100 degrees C is determined, whereby the viscosity ratio uniquely determines the weight of the oil. For example, point 116 of Figure 1 having a viscosity ratio, numerically, of, approximately, 3.2 determines that the weight of oil is 15W. This determines the correct entries in the first and second look-up tables yielding, in this case, a soot compensation factor of, for example, 0.012 from the first look-up table and the best fit line, equation (1), from the second look-up table. At a later time, the soot contamination level (ie., soot percent, or soot %) is measured and a second ratio of the viscosity (second viscosity ratio) measured at 60 degrees C and at 100 degrees C is determined. The second viscosity ratio and soot compensation factor determine a soot compensated viscosity ratio. For example, point 114 having a viscosity ratio, numerically, of, approximately, 3.095, has a soot compensated viscosity ratio, numerically, of, approximately, 3.047 at point 114', as previously described. From equation (1) the soot compensated viscosity ratio yields the fuel dilution level in percent. For example, the soot compensated viscosity ratio of, approximately, 3.047 at point 114' yields a fuel dilution level of 4% using equation (1) corresponding to the actual fuel dilution level in percent of point 114 in Figure 1. Subsequent fuel dilution levels are obtained in a similar manner.

Referring now to the second preferred embodiment of the present invention, Figure 5 is a plot 500 of viscosity differences versus fuel dilution levels in percent for various soot percents, wherein each viscosity difference is the difference between the viscosity at 60 degrees C and the viscosity at 80 degrees C at a particular soot percent and a particular fuel dilution level. Viscosity differences 502 correspond to a soot contamination of 5%. Viscosity differences 504 correspond to a soot contamination of 4%. Viscosity differences 506 correspond to a soot contamination of 3%. Viscosity differences 508 correspond to a soot contamination of 2%. Viscosity differences 510 correspond to a soot contamination of 1%. Viscosity differences 512 correspond to a soot contamination of 0%.

Figure 6 is a graph 600 of ratios of viscosity differences versus fuel dilution levels for various soot percents, wherein the ratios of viscosity differences are the ratios of the viscosity differences 502 through 512 of Figure 5 at a given fuel dilution level to the viscosity difference at 0% soot at the same given fuel dilution level of Figure 5. Figure 6 shows that each of the ratios of the viscosity differences 602 through 612 are almost constant for same level of soot contamination (soot percent, or soot %) independent of the fuel dilution level. Therefore, the ratios of the viscosity differences 502 through 512 of Figure 5 can be compensated for the error introduced by soot contamination by using the soot percent to do the compensation.

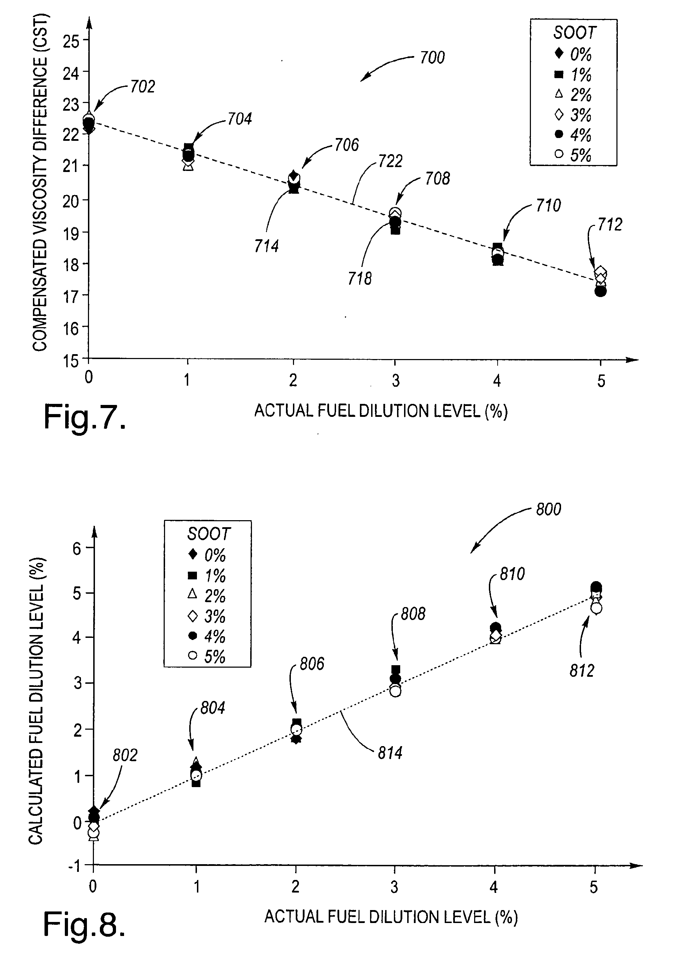

A graph 700 of the soot compensated viscosity differences 702 through 712 of the viscosity differences 502 through 512 of Figure 5 are shown in Figure 7. Each of the viscosity differences 502 through 512 of Figure 5 may be soot compensated to yield soot compensated viscosity differences 702 through 712 by dividing the viscosity difference 502 through 512 at a given soot contamination level (ie., soot percent, or soot %) by the average ratio of the viscosity difference at the same given soot contamination level. For example, the average ratio of the viscosity difference at 5% soot is, approximately, 1.30, numerically, from Figure 6. Point 514 of Figure 5 has a viscosity difference of, approximately, 27, numerically, at 5% soot and a fuel dilution level of 2%. The ratio of 27/1.30 is 20.8 which is the soot compensated viscosity difference at point 714 of Figure 7, corresponding to the viscosity difference of 27 at the point 514 of Figure 5. The average of the ratios of the viscosity differences 602 through 612 at a given soot contamination level (ie., soot percent, or soot %) is the soot compensation factor for the given weight of oil for the given soot contamination level. For example, the average of the ratios of the viscosity differences 602, approximately 1.30, numerically, is the soot compensation factor for 5% soot for the given weight of oil, 15W40 in this case, whereas the average of the ratios of the viscosity differences 604, approximately 1.20, numerically, is the soot compensation factor for 4% soot for the given weight of oil, 15W40 in this case.

In the example of Figure 7, the equation of the straight line 722 is:

$$A = -1.0109B + 22.688 \qquad (2)$$

where A is the fuel dilution level in percent and B is the average soot compensated viscosity difference for a given soot contamination.

Figure 8 is a graph 800 of calculated fuel dilution levels 802 through 812 using equation (2) versus actual fuel dilution levels. The straight line 814 represents the best fit through the points 802 through 812.

The average of the ratios of the viscosity differences 602 through 612 for a given soot contamination level (ie., soot percent, or soot %), the soot compensation factor, and the best fit straight line 722, equation (2), are unique for the given weight of oil 15W40 in the example of the depicted figures. Each different weight of oil will have a unique soot compensation factor for a given soot contamination level and unique best fit straight line. This is due to the dependence of the activation energy characterizing the temperature dependence of different fluids on their molecular weight, as previously described. For each different weight of oil of interest, the soot compensation factors for each given soot contamination level and best fit straight lines can be empirically determined and stored, for example in a vehicle sensor memory or a vehicle microprocessor memory, in third and fourth look-up tables, respectively.

The second preferred embodiment of the present invention may be implemented as follows.

With fresh, clean oil in the engine, a viscosity measurement is made at a predetermined temperature, which measurement is indicative of the weight of the oil. Alternatively, a first ratio of the viscosity (first viscosity ratio) measured at 60 degrees C and at 100 degrees C is determined, whereby the viscosity ratio uniquely determines the weight of the oil. This determines the correct entries in the third and fourth look-up tables.

At a later time, the soot contamination level (ie., soot percent, or soot %) is measured and a difference of the viscosity (second viscosity difference) measured at 60 degrees C and at 80 degrees C is determined. The soot contamination level determines the soot compensation factor for the given weight of oil, 15W40 in this case, from the third look-up table. For example, point 518 of Figure 5 having a viscosity difference, numerically, of, approximately, 25.5, has a soot compensation factor, of, approximately, 1.30, numerically, from Figure 6, as previously described, and produces a soot compensated viscosity difference of, approximately, 19.6, numerically at point 718 of Figure 7. Equation (2), in the fourth look-up table, using the soot compensated viscosity difference yields the fuel dilution level in percent. For example, the soot compensated viscosity difference of, approximately, 19.6, numerically, at point 718 yields a fuel dilution level of 3% using equation (2) corresponding to the actual fuel dilution level in percent of point 518 in Figure 5. Subsequent fuel dilution levels are obtained in a similar manner.

While the invention has been described in connection with what is presently considered to be the most practical and preferred embodiment, it is to be understood that the invention is not to be limited to the disclosed embodiments but, on the contrary, is intended to cover various modifications and equivalent arrangements included within the spirit and scope of the appended claims, which scope is to be accorded the broadest interpretation so as to encompass all such modifications and equivalent structures as is permitted under the law.

## Claims

1. A method for determining fuel dilution of diesel engine lubricating oil in a diesel engine, comprising the steps of:

   providing a first table of first data, said first data comprising a soot compensation factor (102-112), respectively, for each weight of oil selected among a predetermined range of oil weights;
   providing a second table of second data, said second data comprising fuel dilution levels (102'-112') for a plurality of predetermined compensated viscosity ratios;
   determining the weight of the oil in the engine;
   measuring a first viscosity of the oil at a first temperature;
   measuring a second viscosity of the oil at a second temperature;
   determining a ratio (116) of the first and second viscosities;
   compensating for soot in the oil using a said soot compensation factor which is respective of the oil to thereby provide a compensated viscosity ratio (220); and
   comparing the compensated viscosity ratio with said second data to thereby determine the fuel dilution level (302-312).

2. The method of Claim 1, wherein said step of compensating comprises:

   determining a soot percent of the oil;
   obtaining a soot compensation factor (102-112) from the first data responsive to the weight of the oil;
   multiplying the soot compensation factor by the soot percent to obtain a compensation value; and
   subtracting the compensation value from the ratio to provide the compensated viscosity ratio (220).

3. The method of Claim 2, wherein said step of determining the weight of the oil comprises measuring, when the oil is fresh and clean, viscosity of the oil at a predetermined temperature.

4. The method of Claim 2, wherein said step of determining the weight of the oil comprises:

   measuring, when the oil is fresh and clean, a first fresh viscosity of the oil at a first temperature;
   measuring, when the oil is fresh and clean, a second fresh viscosity of the oil at a second temperature; and
   determining a fresh ratio of the first and second fresh viscosities;
   wherein the fresh ratio uniquely determines the weight of the oil.

5. The method of Claim 2, wherein said first and second temperatures are preselected so that any viscosity modifiers in the oil are substantially inactive at the first and second temperatures.

6. The method of Claim 5, wherein said step of determining the weight of the oil comprises measuring, when the oil is fresh and clean, viscosity of the oil at a predetermined temperature.

**7.** The method of Claim 5, wherein said step of determining the weight of the oil comprises:

measuring, when the oil is fresh and clean, a first fresh viscosity of the oil at a first temperature;
measuring, when the oil is fresh and clean, a second fresh viscosity of the oil at a second temperature; and
determining a fresh ratio of the first and second fresh viscosities;
wherein the fresh ratio uniquely determines the weight of the oil.

**8.** A method for determining fuel dilution of diesel engine lubricating oil in a diesel engine, comprising the steps of:

providing a first table of first data, said first data comprising a plurality of soot compensation factors (602-612), respectively, for each soot percent of a predetermined range of soot percents for each weight of oil selected among a predetermined range of oil weights;
providing a second table of second data, said second data comprising fuel dilution levels (702-712) for a plurality of predetermined compensated viscosity differences;
determining the weight of the oil in the engine;
measuring a first viscosity of the oil at a first temperature;
measuring a second viscosity of the oil at a second temperature;
determining a difference between the first and second viscosities;
compensating for soot in the oil using a said soot compensation factor which is respective of the oil and soot percent to thereby provide a compensated viscosity difference (714); and
comparing the compensated viscosity difference with said second data to thereby determine the fuel dilution level (702-714).

**9.** The method of Claim 8, wherein said step of compensating comprises:

determining a soot percent of the oil;
obtaining a soot compensation factor (602-612) from the first data responsive to the weight of the oil and the soot percent; and
dividing the viscosity difference by the soot compensation factor to provide the compensated viscosity difference.

**10.** The method of Claim 9, wherein said step of determining the weight of the oil comprises measuring, when the oil is fresh and clean, viscosity of the oil at a predetermined temperature.

**11.** The method of Claim 9, wherein said step of determining the weight of the oil comprises:

measuring, when the oil is fresh and clean, a first fresh viscosity of the oil at a first temperature;
measuring, when the oil is fresh and clean, a second fresh viscosity of the oil at a second temperature; and
determining a fresh ratio of the first and second fresh viscosities;
wherein the fresh ratio uniquely determines the weight of the oil.

**12.** The method of Claim 9, wherein said first and second temperatures are preselected so that any viscosity modifiers in the oil are substantially inactive at the first and second temperatures.

**13.** The method of Claim 12, wherein said step of determining the weight of the oil comprises measuring, when the oil is fresh and clean, viscosity of the oil at a predetermined temperature.

**14.** The method of Claim 12, wherein said step of determining the weight of the oil comprises:

measuring, when the oil is fresh and clean, a first fresh viscosity of the oil at a first temperature;
measuring, when the oil is fresh and clean, a second fresh viscosity of the oil at a second temperature; and
determining a fresh ratio of the first and second fresh viscosities;
wherein the fresh ratio uniquely determines the weight of the oil.

Fig.1.

Fig.2.

Fig.3.

Fig.4.

Fig.5.

Fig.6.

Fig.7.

Fig.8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6810717 B2

- US 20040036487 A1

### Non-patent literature cited in the description

- **W. J. MOORE.** Physical Chemistry. Addison-Wesley, 1964